Europäisches Patentamt

European Patent Office

Office européen des brevets

(11) Publication number: **0 063 949**
**B1**

(12) **EUROPEAN PATENT SPECIFICATION**

(45) Date of publication of patent specification: 20.05.87

(51) Int. Cl.⁴: **C 12 N 15/00,** C 12 P 21/00, C 12 N 1/00, A 01 N 63/02 // C12R1/07

(21) Application number: 82302137.3

(22) Date of filing: 26.04.82

(54) Plasmids coding for B. Thuringiensis crystal protein, their production and their use in creating genetically engineered bacterial strains, the aforesaid strains per se and their use in producing said protein, said protein so-produced, insecticidal formulations comprising the same and a method of producing an insecticidal effect.

(30) Priority: 27.04.81 US 257963
12.11.81 US 320560
30.03.82 US 362634

(43) Date of publication of application:
03.11.82 Bulletin 82/44

(45) Publication of the grant of the patent:
20.05.87 Bulletin 87/21

(84) Designated Contracting States:
AT BE CH DE FR GB IT LI LU NL SE

(56) References cited:
PROC. NATL. ACAD. SCI. USA, vol.78, no.5,
May 1981, (US), H.E. SCHNEPF et al.: "Cloning
and expression of the Bacillus thuringiensis
crystal protein gene in Escherichia coli", pages
2893-2897

CHEMICAL ABSTRACTS, vol.93, no.5, August
4, 1980, page 481, abstract no.41304r,
Columbus, Ohio (US), A.W.M. PHYLLIS et al.:
"Genetic manipulation in the insect pathogen
Bacillus Thuringiensis"

(73) Proprietor: Washington Research Foundation
1107 N.E. 45th Street, Suite 322
Seattle Washington 98195 (US)

(72) Inventor: Schnepf, Harry Ernest
4033 Eastern Avenue North
Seattle Washington 98103 (US)
Inventor: Whiteley, Helen Riaboff
13244 40th Avenue N.E.
Seattle Washington 98125 (US)

(74) Representative: Bizley, Richard Edward et al
BOULT, WADE & TENNANT 27 Furnival Street
London EC4A 1PQ (GB)

(56) References cited:
CHEMICAL ABSTRACTS, vol.92, no.5, February
4, 1980, page 409, abstract no.37544f,
Columbus, Ohio (US), A.F. KLIER et al.:
"Structure of cloned ribonomal DNA cistrons
from Bacillus thuringiensis"

GENES, John Wiley & Sons, 1983, p. 306-309

## Description

The invention describes novel genetically engineered plasmids. The disclosure describes plasmids that have been deposited with the American Type Culture Collection, Rockville, MD 20852, under the Budapest Treaty. Plasmid pES1 is carried in *Escherichia coli* strain ES12; the strain and plasmid have been assigned ATCC Number 31995. Plasmid pJWK20 is carried in *Escherichia coli* strain JWK1, the strain and plasmid have been assigned ATCC Number 31997. Plasmid pJWK18 is carried in *Escherichia coli* strain JWK11; the strain and plasmid have been assigned ATCC Number 31998.

This invention relates generally to the production of substances for the control of insects injurious to certain plants. More particularly, the invention relates to an improved means for producing substances toxic to larvae of the tobacco hornworm *Manduca sexta* and related species.

As is well known, the crystal protein made by *B. thuringiensis* is toxic to the larvae of a number of lepidopteran insects. Preparations containing crystals are used commercially as a highly selective biological insecticide. However, problems connected with the use of such insecticides, together with relatively high manufacturing costs, have made it difficult, in many cases, for such insecticides to compete effectively with other commercially available products.

The fact that *B. thuringiensis* produces the crystal protein only during sporulation represents a significant disadvantage in connection with the manufacture and use of the crystals. Such a growth phase limitation, particularly in an industrial process, can result in inconvenience and excessive time requirements during manufacture. In fact, certain pressures resulting from growth phase limitations or other factors may even result in strains of *B. thuringiensis* losing their ability to produce the crystals. Such acrystalliferous strains do not have insecticidal activity.

The present invention discloses and embraces plasmids (and their manufacture) that are capable of replication in an *E. coli* bacterial host species and that contain expressible heterologous DNA encoding for the crystal protein of *B. thuringiensis*.

Thus, the invention provides a plasmid containing DNA derived from a plasmid fraction of *B. thuringiensis* of molecular mass greater than $10 \times 10^6$ $M_r$ and identifiable with a DNA fragment probe derived from plasmid pES1 (obtainable from ATCC Number 31995) which probe is contained within two Pvu II cleavage sites thereon, which plasmid is capable of replication in an *E. coli* bacterial host species and contains expressible heterologous DNA coding for the crystal protein of *Bacillus thuringiensis* and includes an expression mechanism for said heterologous DNA which is recognized by the *E. coli* bacterial host species system but does not exhibit substantial growth phase limitations in the *E. coli* bacterial host species. In another aspect, the invention comprises generically engineered *E. coli* bacterial strains transformed by such plasmids and, in a further aspect, the manufacture or creation of such strains.

*E. coli* bacterial strains genetically engineered to contain the recombinant plasmids of the present invention can be produced by transformation and can express *B. thuringiensis* crystal protein. A method of preparing the protein by growing such strains is included in the invention. The protein produced by these genetically engineered strains is toxic to the larvae of a number of lepidopteran insects. Also included in the invention is a method of producing an insecticide in an environment using such protein, preparations or formulations in a standard manner or by exposing the environment to growth of a genetically engineered strain as aforesaid.

The invention, of course, provides a method of producing a plasmid of the invention which comprises isolating from a plasmid fraction of a crystal protein-producing strain of *Bacillus thuringiensis*, which fraction is of molecular mass greater than $10 \times 10^6$ $M_r$, a fragment of DNA comprising an expressible DNA coding for the crystal protein, said fragment being identifiable with a DNA probe from plasmid pES1 (obtainable from ATCC Number 31995) which probe is contained within two Pvu II cleavage sites thereon, and ligating said DNA fragment with DNA of a plasmid capable of replication in an *E. coli* bacterial species whereby the correct arrangement for expression of the heterologous crystal protein-producing DNA coding in said *E. coli* bacterial host is achieved with the expression mechanism recognized by the host without exhibiting substantial growth phase limitations.

As indicated earlier, the invention also includes a method of creating a genetically engineered bacterial strain which is capable of producing the crystal protein of *Bacillus thuringiensis* comprising transforming an E. coli bacterial strain by introducing thereinto a plasmid of the invention whereby the expressible genetic material in the host includes heterologous DNA coding for the crystal protein of *B. thuringiensis*.

In accordance with a preferred form of the invention, novel recombinant plasmids are created when the known cloning vector plasmid pBR322, Bolivar et al, *Gene* 2:95-113 (1977), is combined with plasmid fragments obtained from plasmids harbored by strains of *B. thuringiensis*. The "large plasmid fraction" is especially preferred. This "large plasmid fraction" includes fragments having a size greater than about 10 megadaltons. The plasmids harbored by strains of *B. thuringiensis* are believed to be responsible for the production of the crystal protein in *B. thuringiensis*. The *B. thuringiensis* plasmid fragments used to construct the recombinant crystal protein coding plasmids of the present invention can be derived from a variety of *B. thuringiensis* strains, some of which are publicly available. For example, *B. thuringiensis* variety *Kurstaki* HD-1 is available from the Agricultural Research Culture Collection (NRRL) in Peoria, Illinois, U.S.A. It has been awarded NRRL Number B-3792. *B. thuringiensis* variety *Kurstaki* HD-73 is also available from the Agricultural Research Culture Collection. It has been awarded NRRL Number B-4488. In the Examples given hereinafter the use of several *B. thuringiensis* strains is illustrated. Such Examples are included for illustrative purposes only and are not intended to limit the scope of the invention to use of fragments derived from such strains.

The invention will now be further described and illustrated by the following Examples, and by reference to the accompanying drawings, wherein:—

Figure 1 is an agarose gel analysis of plasmid DNAs and marker DNA fragments comprising a photograph of 0.7% (lanes 1—5) and 0.35% (lanes 6 and 7) agarose slab gels stained with ethidium bromide;

Figure 2 is a hybridization analysis of plasmid DNAs transferred to nitrocellulose comprising a photograph of a 0.7% ethidium bromide stained agarose gel (lanes 1—3) and autoradiograms of $^{32}$P labeled plasmids (lanes 4—12); and

Figure 3 is a photograph showing a radioimmune assay of crystal preotein and proteins produced by bacterial strain ES12 before and after digestion with trypsin; and

Figure 4 is a photograph of an ethidium bromide stained gel showing plasmids detected in extracts of different strains of *B. thuringiensis.*

Example IV, given hereinafter, is a survey of *B. thuringiensis* strains for plasmid content and the ability of these plasmids to hybridize with a particular genetic probe for identification of the crystal protein gene.

Example I

Use of *B. thuringiensis* variety *kurstaki* HD-1-Dipel plasmid DNA fragments in the construction of recombinant plasmids in accordance with the present invention.

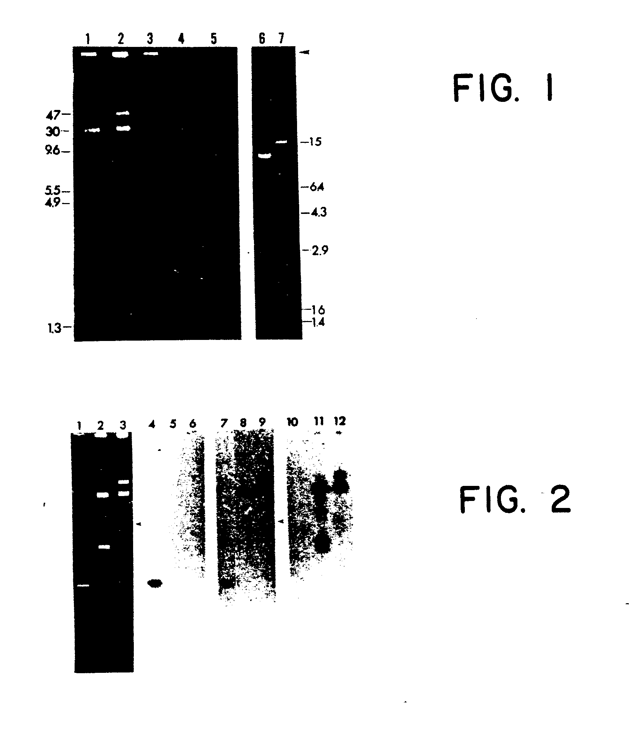

Crystal-producing strains of *B. thuringiensis* vary in the number and sizes of the plasmids they contain. Plasmids obtained from *B. thuringiensis* variety *kurstaki* HD-1-Dipel range in molecular mass from approximately 47 to 1.32 megadaltons as shown in lane 1 of Figure 1, which shows the total plasmid complement normally obtained from this strain. Larger plasmids are present in this strain but were not readily detected as closed circular forms under the conditions of growth used in this study. However, plasmid fractions isolated from *B. thuringiensis* contained small amounts of the linearized forms of these large plasmids. Two plasmid fractions were obtained from the *B. thuringiensis* variety *kurstaki* HD-1-Dipel strain, as shown in lanes 2 and 3 of Figure 1. One fraction, shown in lane 2, contained the 47, 32 and 30 megadalton plasmids as well as trace amounts of linearized forms of the very large plasmids of *B. thuringiensis* (molecular weight×$10^6$ shown on left). The second fraction contained smaller plasmids of 4, 9, 5.2, 5.5 and 9.6 megadaltons, as shown in lane 3.

A preferred recombinant plasmid of the invention, designated pES1 (ATCC Number 31995), is shown in lane 4 of Figure 1 in which the gel analysis of the plasmid may be compared with pBR322, shown in lane 5. After digestion by an enzyme which cuts the plasmid once, the linearized plasmid had a mobility corresponding to ca. 11×$10^6$ M$_r$ when compared to *Hind* III digested lambda DNA (shown in lanes 6 and 7 of Figure 1). Molecular weights (×$10^{-6}$) on the right of the Figure refer to the *Hind* III digest of lambda DNA. The arrow on the right in Figure 1 marks the origin for lanes 6 and 7.

In constructing recombinant plasmids, such as pES1 (ATCC Number 31995) according to the invention, the following detailed procedures were followed. These procedures are also outlined in Schnepf and Whiteley, *Proc. Nat. Acad. Sci.,* USA, 78:2893—2897 (1981). They are given only by way of example and are not intended to limit the scope of the claims herein.

The two separated plasmid fractions, designated as "large" and "small", obtained from *B. thuringiensis* var. *kurstaki* HD-1-Dipel were digested with varying dilutions of *Sau*3A1. This was monitored by agarose gel electrophoresis. To generate a source of inserts, the "large plasmid fraction" was digested with the minimum amount of enzyme needed to convert all the closed covalent circular molecules to the linear form. Restriction endonucleases *Sal* I, *Hind* III, *Bam*H 1 and *Sau*3A1 were used as recommended by the manufacturer (New England Biolabs). This resulted in fragments with an average size greater than 10 megadaltons. For the "small plasmid fraction", an amount of *Sau*3A1 was used which left some closed covalent circular molecules, but produced few linear fragments under 2×$10^6$ M$_r$ in size.

Each fraction was then ligated to pBR322 that had been opened by digestion with the restriction enzyme *Bam* HI. Plasmid pBR322 was isolated from *E. coli* strain HB101 (pBR322) as outlined by Blair et al *Proc. Nat. Acad. Sci.,* USA, 69:2518—2522 (1972). Plasmid pBR322 is on deposit with ATCC, is publicly available and its accession number is ATCC 31071. DNA fragments (3 ug of *B. thuringiensis* plasmid DNA and 0.15 ug of pBR322 DNA in a volume of 10 ul) were ligated as described for cohesive ends by Maniatis et al in *Cell* 15:667—701 (1978).

Recombinants were then screened and selected. *Staphylococcus* Protein A (Pharmacia) was labeled with $^{125}$I (Amersham) using chloramine T as described by Erlich et al, *Cell* 13:681—689 (1978). The DNA polymerase I-catalysed fill-in reaction described by Maniatis et al *Proc. Nat. Acad. Sci.,* USA, 72:1184—1188 (1975) was used to label DNA fragments of *Sau*3A1 digests with [a-$^{32}$P]dCTP (Amersham).

Plasmids were obtained from *B. thuringiensis* var. *kurstaki* HD-1-Dipel, kindly provided by Dr. Lee A. Bulla, Jr., according to the method for plasmid screening of White and Nester, *J. Bacteriol.* 141:1134—1141 (1980). All plasmid preparations were additionally purified by centrifugation in cesium chloride-ethidium bromide gradients. Samples (100 ug DNA per gradient) for cloning experiments were fractionated by centrifugation through 5—25% sucrose gradients for 2.5 hours at 35,000 RPM in an International B-60 centrifuge using the SB-283 rotor. Electrophoresis in agarose gels was used to analyze plasmid DNAs as described by Meyers et al, *J. Bacteriol.* 127:1529—1537 (1976). Fragments of the plasmids were produced by digestion of DNA with restriction enzymes as is known in the art. Hybridization to plasmid DNAs was performed after partial depurination as described by Wahl et al, *Proc. Nat. Acad. Sci.,* USA, 76:3683—3687 (1979) and transfer of DNA from gels to nitrocellulose was done as described by Thomashow et al *Cell* 19:729—739 (1980).

The recombinant plasmids thus produced were then transformed into *E. coli*. Transformation was carried out as described by known procedures and transformants were selected on media containing 100 ug/ml ampicillin. Since cloning was performed by insertion of messenger DNA into the *Bam*H 1 site of pBR322, which is located in a gene coding for tetracycline resistance, ampicillin resistant transformants were screened for sensitivity to tetracycline (25 ug/ml). Colonies resistant to ampicillin but sensitive to tetracycline were presumed to contain inserts.

Those transformed colonies presumed to carry *B. thuringiensis* DNA inserts were then screened for the production of crystal protein antigen using antibodies and $^{125}$I-Protein A. To prepare antibodies to the crystals, the crystals were first purified from sporulated cultures of *B. thuringiensis* grown in modified G medium, Aronson et al, *J. Bacteriol.* 106:1016—1025 (1971), by four successive centrifugations in Renograffin (Squibb) gradients. Contamination with spores was estimated at less than 0.1% by phase microscopy. Solubilized crystals were electrophoresed on preparative 10% polyacrylamide slab gels containing NaDodSo$_4$, and the portion of the gel containing the major crystal protein polypeptide was sliced from the gel, crushed, mixed with an equal amount of Freund's Complete Adjuvant and used to immunize rabbits. The immunoglobulin G fraction was purified from the serum of the immunized rabbits by precipitation with ammonium sulfate and chromatography on DEAE cellulose and analyzed by Ouchterlony immunodiffusion, as is known in the art.

To screen by detection of antigens, colonies were first transferred from agar plates to filter paper and denatured with phenol-chloroform-heptane and chloroform-methanol as described by Henning et al, *Anal. Biochem.* 97:153—157 (1979). The filters were soaked in 1% bovine serum albumen (Sigma, fraction V) and incubated with antibody and $^{125}$I-Protein A as described by Renart et al, *Proc. Nat. Acad. Sci.,* USA, 76:3116—3120, (1979). Colonies containing material capable of reacting with the crystal protein antibodies were detected by autoradiography. The concentrations of antibody ($10^{-3}$ to $10^{-4}$ dilution) and $^{125}$I-Protein A (0.2 to $1 \times 10^6$ cpm) were varied to obtain conditions permitting the detection of 5 ng crystal protein in 1 ul spotted on a filter while colonies of *E. coli* HB101 (pBR322) were either unreactive or appeared light against a grey background. Protein samples were electrophoresed on 10% NaDodSO$_4$/polyacrylamide slab gels, transferred electrophoretically to nitrocellulose, and incubated with antibody ($5 \times 10^{-3}$ dilution) and $^{125}$I-Protein A ($6 \times 10^5$ cpm) in accordance with known procedures. The reaction of transferred peptides with antibody and $^{125}$I-Protein A was detected by radioautography.

Cells grown for 16 hours in L broth containing 100 ug/ml ampicillin were harvested by centrifugation, suspended in 0.1 M Tris buffer at pH 7.0, 1 mM EDTA, 200 ug/ml phenylmethylsulfonyl fluoride and disrupted by sonication. Insoluble material, obtained by centrifugation of the sonicates at 100,000×g for 30 minutes, was thoroughly suspended in 4M urea, 0.285M 2-mercaptoethanol, 0.05M NaHCO$_3$, pH 9.5, and dialysed against 0.05M Tris buffer pH 7.4 for insect toxicity assays or for electrophoretic analysis. For proteolysis with subtilisin or trypsin, the samples were prepared as above substituting 0.05M cyclohexylaminoethane sulfonic acid (CHES), pH 10.0, 0.285M 2-mercaptoethanol for pH 9.5 urea buffer; final dialysis was against 0.01M CHES buffer, pH 10.0. For trypsin digestion, 0.01M CHES was used as the buffer and digestion was carried out at room temperature during dialysis against that buffer; digestion with subtilisin was performed according to Cleveland et al, *J. Biol. Chem.* 252:1102—1106 (1977). The reactions were stopped by boiling the samples in electrophoresis sample buffer and polypeptides capable of reacting with antibody to the crystal protein were detected using $^{125}$I-Protein A and radioautography as described above. Protein concentrations were determined according to Bradford, *Anal. Biochem.* 72:248—254 (1976).

The recombinant plasmid pES1 (ATCC Number 31995) consists of the plasmid vector pBR322 and DNA homologous to the 30, 32 and 47 megadalton plasmids, as well as DNA homologous to linearlized forms of the very large plasmids of *B. thuringiensis*. One explanation for this result is that several *Sau*3A1 partial digest fragments from each of the large *B. thuringiensis* plasmids, were ligated together; this heteromultimer could then have been ligated into the vector. Another possibility is that the apparent hybridization to the 47 megadalton plasmid as shown in Figure 2, is actually a trailing artifact from the 30 or 32 megadalton plasmids.

Example II

Evidence that *E. coli* transformed by recombinant plasmids of the present invention produce *B. thuringiensis* crystal protein.

The recombinant plasmids of the present invention can be used to transform *E. coli* cells. These transformed cells produce *B. thuringiensis* crystal protein. Such transformation is possible because the recombinant plasmids of the present invention contain both vector DNA and DNA from *B. thuringiensis* plasmids that codes for the crystal protein. Plasmid pES1 (ATCC Number 31995) was used to demonstrate this.

As illustrated in Figure 2, plasmid pES1 (ATCC Number 31995) contains substantial homology with DNA from the plasmid pBR322 as well as DNA homologous to the 30, 32 and 47 megadalton plasmids as well as DNA homologous to linearized forms of the very large plasmids of *B. thuringiensis*. In Figure 2, lanes 1—3 show a photograph of a 0.7% ethidium bromide-stained agarose gel: Lane 1 shows *Bam*H 1 digested pBR322 DNA; lane 2 shows total plasmid complement of *B. thuringiensis* and lane 3 shows the "large plasmid fraction" from *B. thuringiensis*. Lanes 4—6 show an autoradiogram of $^{32}$P-labeled pBR322 DNA hybridized with: *Bam*H 1 digested pBR322 DNA, as shown in lane 4; total plasmid complement, as shown in lane 5; and the "large plasmid fraction" from *B. thuringiensis,* as shown in lane 6. Lanes 7—9 show an autoradiogram of $^{32}$P-labeled pES1 (ATCC Number 31995) DNA hybridized with: *Bam*H 1 digested pBR322, as shown in lane 7; total plasmid complement, as shown in lane 8; and "the large plasmid fraction" from *B. thuringiensis,* as shown in lane 9. Lanes 1—12 show an autoradiogram of $^{32}$P-labeled total *B. thuringiensis* plasmid DNA hybridized with: *Bam*H 1 digested pBR322 DNA as shown in lane 10; total plasmid complement, as shown in lane 11; and the "large plasmid fraction" from *B. thuringiensis,* as shown in lane 12. The arrows in this Figure show the position of the linearized fragments and the hybridization of the probe to such fragments.

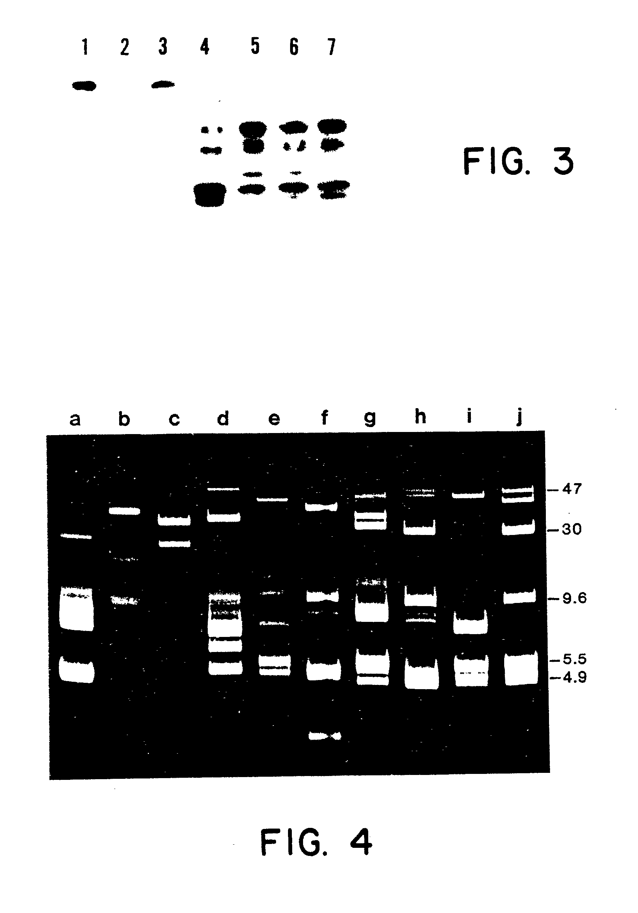

Figure 3 presents a radioimmune assay of crystal protein produced by transformed *E. coli* strain ES12 before and after digestion with trypsin.

Autoradiograms of a solid-phase radioimmune assay for polypeptides are shown in lanes 1—3 of Figure 3. These fragments react with anti-crystal antibody following NaDodSO$_4$/polyacrylamide gel electrophoresis. Lane 1 shows 1 ug *B. thuringiensis* crystal protein. Lane 2 shows 100 ug *E. coli* HB101 (pBr322) protein extract. Lane 3 shows 100 ug ES12 protein extract. Lane 4 is derived from dissolved *B. thuringiensis* crystals reacted with 5% (wt/wt) trypsin at pH 10 for 3 hours at room temperature. Lanes 5—7 show the ES12 extract reacted with: 2% (wt/wt) trypsin, as shown in lane 5; 4% (wt/wt) trypsin, as shown in lane 6; and 6% (wt/wt) trypsin at pH 10 for 3 hours at room temperature, as shown in lane 7.

Lanes 1—3 of Figure 3 show that the antigen made by ES12, which reacted with crystal protein antibodies, had the same electrophoretic mobility as the *B. thuringiensis* crystal protein. Dissolved *B. thuringiensis* crystals and cell extracts of HB101 (pBR322) and ES12 were electrophoresed on a NaDodSO$_4$/polyacrylamide gel and reacted with anti-crystal antibody and $^{125}$I-Protein A after transfer to nitrocellulose. The ES12 extract, shown in lane 3 of Figure 3, contained a polypeptide antigen having the same (or very similar) electrophoretic mobility as the dissolved *B. thuringiensis* crystals shown in lane 1 of Figure 3. This polypeptide antigen was missing from a similar extract of HB101 (pBR322), as shown in lane 2 of Figure 3, and was not detected when pre-immune serum was substituted for anti-crystal antibody, or when $^{125}$I-Protein A was used without prior antibody treatment (data not shown).

Comparison of the bands shown in Figure 3 (1 ug crystal protein in lane 1) with the total amount of protein applied to lane 3 (100 ug) indicates that the crystal protein antigen accounts for a small amount of the protein (1% or less) in ES12. When the radioimmune detection of polypeptide was used to monitor the fractionation of ES12 extracts it was found that a reducing agent plus a denaturant or an alkali pH was required to solubilize the crystal protein antigen. These conditions are also required to solubilize *B. thuringiensis* crystals.

It has been reported by Lilley et al, *J. Gen. Microbiol.* 118:1-11 (1980) that the crystal protein can be digested by a number of proteases at pH 10 to produce primarily a single polypeptide. Lanes 4—7 of Figure 3 show the results of an experiment where dissolved *B. thuringiensis* crystals and an eluate from the particulate fraction of ES12 were subjected to partial digestion at pH 10 with the indicated amounts of trypsin. As seen in lanes 5, 6, and 7 of Figure 3, digestion of the ES12 extract with increasing amounts of trypsin yielded a pattern, as shown in lane 7 of Figure 3, which was similar to the pattern produced by trypsin digestion of the crystal protein of *B. thuringiensis,* as shown in lane 4 of Figure 3. Qualitatively the patterns of the bands generated from the two preparations were similar. The quantitative, and minor qualitative, differences may reflect a less efficient digestion of the crystal protein antigen in ES12 extracts due to the presence of numerous other polypeptide species.

The larger number of polypeptides produced by trypsin digestion of the crystal protein in these experiments as opposed to the number reported by Lilley et al, *supra,* may be due to differences in the conditions of trypsin treatment. Similar experiments using subtilisin also showed agreement in the electrophoretic mobilities of the bands produced from the crystal protein of *B. thuringiensis* was obtained by assaying for insect toxicity. Extracts of particulate fractions obtained from *E. coli* HB101 (pBR322) and ES12 were mixed with feed meal supplied to neonate caterpillars of the tobacco hornworn *Manduca sexta.* Neonate larvae of the *Manduca sexta* were supplied by Drs. J. Truman and L. Riddiford, Department of Zoology, University of Washington. Extracts were prepared as described above from 8 liters of the appropriate *E. coli* strains; 6—8 mls of extract were mixed with 50 ml of molten agar-based diet and quickly poured to give a shallow layer. Strips of the solidified diet were placed in glass vials (3—4 ml/vial) with one neonate larva for 10 days at room temperature.

The results indicated that the extracts of the genetically engineered recombinant strain were toxic to caterpillars. The 15 larvae exposed to the ES12 extracts did not complete the first instar before death. An equivalent amount of extract from control strain, *E. coli* HB101 (pBR322), had no noticeable effect on the growth and development of 15 larvae through at least the third instar when compared to larvae grown on feed meal without any extract added. Identical results were obtained when this experiment was repeated with another set of *E. coli* extracts. The minimal amount of extract of ES12 required to kill the caterpillar larvae has not yet been determined. Assuming that the crystal protein antigen in the ES12 extracts was 0.5—1% of the total protein, then the feed meal prepared with the extract from ES12 contained 12—25 ug crystal protein per ml whereas a concentration of 2 ug/ml of pure crystal protein is sufficient to achieve 100% killing of the larvae.

The resulting transformed strain of *E. coli*, ES12, carries a recombinant plasmid and produces a protein antigen that reacts with antibodies specific for the crystal protein of *B. thuringiensis*. This is confirmed by the above described tests wherein the recombinant plasmid isolated from this strain, pES1 (ATCC Number 31995) simultaneously transforms cultures of *E. coli* strain HB101 to both ampicillin resistance and the production of the crystal protein antigen, whereas the plasmid vector pBR322 transforms HB101 to ampicillin and tetracycline resistance but no crystal antigen can be detected. Test results indicate that the DNA insert of the recombinant plasmid pES1 (ATCC Number 31995) encodes the ability to make a polypeptide possessing properties similar to those of the crystal protein of *B. thuringiensis*. In fact, the results strongly suggest that the DNA insert of pES1 (ATCC Number 31995) encodes the gene for the crystal protein of *B. thuringiensis* and that this gene is expressed in a biologically active form in *E. coli*. Preparations made from this new genetically engineered strain, ES12, may therefore be used as an insecticide for appropriate cases.

Example III

Use of *B. thuringiensis* var. *kurstaki* HD-73 (NRRL B-4488) plasmid DNA fragments in the construction of recombinant plasmids in accordance with the present invention.

*B. thuringiensis* var. *kurstaki* HD-73 contains fewer so-called large plasmids than do some of the other *B. thuringiensis* strains. However, as this Example illustrates, this strain can be used to generate the DNA fragments utilized in constructing the recombinant plasmids of the present invention. The "large plasmid fraction" from *B. thuringiensis* var. *kurstaki* HD-73 was obtained by sucrose gradient centrifugation in the same way as the "large plasmid fraction" from *B. thuringiensis* var. *kurstaki* HD-1-Dipel described in Example I. Digestion of this fraction with restriction enzyme *Bgl* II yielded approximately six fragments, one of which hybridized with a *Pvu* II-C DNA probe obtained from pES1 (ATCC Number 31995). The probe is discussed more fully in Example IV. The "large plasmid fraction" was digested with *Bgl* II, ligated to *Bam*H I-digested pBR322 and transformed into *E. coli*. Colonies which were ampicillin-resistant and tetracycline-sensitive were screened for hybridization with the *Pvu* II-C DNA probe and for their ability to synthesize the crystal protein antigen. A strain was isolated designated JWK1, having a plasmid, pJWK20 (ATCC Number 31997), whose linearized mass is approximately 13.5 megadaltons. The results of preliminary analysis using *Bgl* II, *Sal* I, and *Hind* III digestion of pJWK20 and the "large plasmid fraction" from HD-73 suggest that pJWK20 has a single insert of approximately 10.9 megadaltons derived from the 45 megadalton plasmid of *B. thuringiensis* var. *kurstaki* HD-73.

Extracts of genetically engineered strain JWK1 were found to contain a polypeptide of approximately 130,000 $M_r$ which reacted with the antibody to the crystal protein from HD-1-Dipel. The mobility of this peptide matched that of the crystal protein from HD-1-Dipel. Tests for insect toxicity showed that feed meal supplemented with extracts of transformed *E. coli* strain JWK1 were lethal for larvae of the tobacco hornworm. The onset of toxic symptoms was similar to that observed for larvae fed with meal containing solubilized crystal protein. As in previously described toxicity tests, meal containing extracts of *E. coli* (pBR322) did not retard the growth of the larvae. Thus it can be seen that genetically engineered *E. coli* strain JWK1 produces a protein which is similar in size, antigenicity and biological activities to the crystal protein of *B. thuringiensis* HD-1-Dipel.

Example IV

Survey of *B. thuringiensis* strains for plasmid content and the ability of these plasmids to hybridize with a probe from pES1 (ATCC Number 31995).

Restriction enzyme analysis of transposon Tn5 inserts into pES1 (data not shown) indicates that a major portion of the presumed crystal protein gene is contained within two *Pvu* II cleavage sites on the *B. thuringiensis* plasmids. The cleavage sites define a *Pvu* II-C DNA fragment that can be used as a probe to analyze plasmid profiles of the various strains of *B. thuringiensis* to determine which plasmids contain the gene. The *Pvu* II-C probe fragment was purified by sucrose gradient sedimentation. The DNA fragments were labeled with alpha-$^{32}$-P-dCTP using either the DNA polymerqse-catalyzed fill-in reaction described by Maniatias et al, *Cell* 15:667—701 (1978), or by nick translation, described by Maniatias et al, *Proc. Nat. Acad. Sci.*, USA 72:1184—1188 (1975). The probe was then hybridized to plasmids from various *B. thuringiensis* strains after electrophoresis and transfer to nitrocellulose. At the time of filing, about twenty crystal-producing strains of *B. thuringiensis* had been examined for plasmid content and the ability of these plasmids to hybridize with the *Pvu* II-C probe fragment from pES1 (ATCC Number 31995). An example of one of the gels run as part of this survey is shown in Figure 4. The photograph is of an ethidium bromide stained gel that shows plasmids detected in extracts of various *B. thuringiensis* strains. Lane a is subspecies tolworthii; lane b is subspecies *darmstadiensis;* lane c is subspecies *sotto*; lanes d-g are var. *thuringiensis* F-10, HD-290, HD-120, and HD-2 respectively; lanes h-j are var. *Kurstaki* HD-244, HD-73 and HD-1, respectively. The numbers in the margin indicate plasmid size in megadaltons. Other gels were run (data not shown) with extracts from *B. thuringiensis* subspecies *alesti* HD-4, subspecies *toumanoffi* F-9, subspecies *galleriae* HD-8, subspecies *wuhnanesis* F-6, and subspecies *morrisoni* F-5. The results of the electrophoresis shown in Figure 4 demonstrated the presence of several plasmid bands in each one. Presumably, most of the bands consist of closed circular molecules although some bands may represent open circular forms. The strains varied greatly in the number and sizes of the plasmids they contained; however some bands of the same or similar mobilities were present in several strains. It is pointed out that the data presented in Figure 4 represent the analysis of routine CsCl-purified plasmid preparations and that no attempts have been made to verify conditions of growth, plasmid extraction or electrophoresis in order to detect all possible plasmids or to duplicate electrophoretic conditions used by other investigators. In addition, the sizes have been roughly estimated by comparisons with the mobilities of plasmids whose contour lengths have been determined by electron microscopy by Stahley et al, *Biochem. Biophys. Res. Comm.,* 84:581—588 (1978).

The strains examined in the experiment shown in Figure 4 differ in flagellar serotype and in their ability to cross-react with the antibody to the crystal protein of strain HD-1-Dipel. The serotype of the *kurstaki* strains is 3a, 3b; the *thuringiensis* strains are type 1; the subspecies *sotto* is type 4a, 4b; subspecies *darmstadiensis* is type 10 and subspecies *tolworthii* is type 9. See De Barjac and Bonnefoi, *C. R. Acad. Sci.,* 264:1811—1813 (1867). Fourteen of the twenty strains tested reacted with the antibody to the crystal protein of strain HD-1-Dipel and showed hybridization with *Pvu* II-C probe.

Example V

Use of *B. thuringiensis* subspecies *sotto* plasmid DNA fragments in the construction of the recombinant plasmids of the present invention.

The prodecures used to clone the crystal protein gene from *B. thuringiensis* subspecies *sotto* are essentially the same procedures used to clone the gene from *B. thuringiensis* var. *Kurstaki* HD-73. The procedures are outlined in Example III.

The subspecies *sotto* was selected for cloning the crystal protein gene because it contains only two plasmids and belongs to a different serotype. To clone the gene, total plasmid DNA purified by centrifugation in a CsCl-ethidium bromide gradient was partially restricted with endonuclease *Mbo* I. The resulting fragments were ligated into the *Bam*H I site of pBR322. After transformation into *E. coli,* colonies were selected which were ampicillin-resistant and tetracycline-sensitive. These colonies were screened for their ability to hybridize with the *Pvu* II-C fragment and for their ability to produce the crystal protein. One recombinant strain, JWK 11, contained the plasmid pJWK18; the size of the inserted *B. thuringiensis* plasmid DNA has not yet been determined accurately. Strain JWK 11 produces a 130,000 M$_r$ protein which cross-reacts with antisera prepared against the crystal protein isolated from *B. thuringiensis kurstaki* HD-1-Dipel. Extracts of JWK 11 are toxic to larvae of *Munduca sexta.*

It may be seen therefore that, in accordance with the invention, crystal protein of *B. thuringiensis* is produced by a host strain transformed with recombinant plasmids. *In vivo, B. thuringiensis* only produces crystal protein during sporulation. An advantage of this invention is the absence of the necessity for the host to sporulate in order for expression of the crystal protein to occur. Expression of the genetic information in the plasmids of the invention is not limited to a specific growth phase in the host because the crystal protein is expressed in accordance with an expression mechanism that is recognized by the host species in all major growth phases. By removing growth phase restrictions, production of the toxin is now possible by continuous culture rather than batch culture. This can permit high rate, high productivity fermentations with a decrease in capital equipment requirements.

Various modifications and variations within the scope of the invention in addition to the precise procedures shown and described herein will become apparent to those skilled in the art from the foregoing description and accompanying drawings.

References

1. Aronson, A. I., Angelo, N., and Holt, S. C., *J. Bacteriol.* 106:1016—1025 (1971).
2. Blair, D. G., Sherratt, D. J., Clewell, D. B., and Helinski, D. R., *Proc. Nat. Acad. Sci.,* USA, 69:2518—2522 (1972).
3. Bolivar, F., Rodriguez, R. L., Greene, P. J., Betlach, M. C., Heyneker, H. L., and Boyer, H. W., *Gene* 2:95—113 (1977).
4. Bradford, M., *Anal. Biochem.* 72:248—254 (1976).
5. Cleveland, D. W., Fischer, S. G., Kirschner, M. W., and Laemmli, U. K., *J. Biol. Chem.* 252(3):1102—1106 (1977).
6. DeBarjac, H., and Bonnefoi, A., *C. R. Acad. Sci.* 264:1811—1813 (1967).
7. Erlich, H. A., Cohen, S. N., and McDevitt, H. O., *Cell* 13:681—689 (1978).
8. Henning, U. Schwarz, H., and Chen, R., *Anal. Biochem.* 97:153—157 (1979).
9. Lilley, M., Ruffell, R. N., and Somerville, H. J. *J. Gen. Microbiol.* 118:1—11 (1980).

10. Maniatis, T., Hardison, R. C., Lacy, E., Lauer, J., O'Connell, C., Quon, D., Sim, G. K., and Efstratiadis, A. *Cell* 15:667—701 (1978).
11. Maniatis, T., Jeffrey, A., and Kleid, D. G., *Proc. Nat. Acad. Sci.,* USA, 72:1184—1188 (1975).
12. Meyers, J. A., Sanchez, D., Elwell, L. P., and Falkow, S., *J. Bacteriol.* 127:1529—1537 (1976).
13. Renart, J., Reiser, J., and Stark, G. R., *Proc. Nat. Acad. Sci.,* USA 76:3116—3120 (1979).
14. Schnepf, H. E., and Whiteley, H. R., *Proc. Nat. Acad. Sci.,* USA 78:2893—2897 (1981).
15. Stahly, D. P., Dingman, D. W., Bulla, L. A., and Aronson, A. I., *Biochem. Biophys. Res. Comm.* 84:581—588 (1978).
16. Thomashow, M. F., Nutter, R., Montoya, A. L., Gordon, M. P., and Nester, E. W., *Cell* 19:729—739 (1980).
17. Wahl, G. M., Stern, M., and Stark, G. H., *Proc. Nat. Acad. Sci.,* USA, 76:3683—3687 (1979).
18. White, F. F., and Nester, E. W., *J. Bacteriol.* 141:1134—1141 (1981).

**Claims for the Contracting States: BE, CH, DE, FR, GB, IT, LI, LU, NL, SE**

1. A plasmid containing DNA derived from a plasmid fraction of *B. thuringiensis* of molecular mass greater than $10 \times 10^6$ $M_r$ and identifiable with a DNA fragment probe derived from plasmid pES1 (obtainable from ATCC Number 31995) which probe is contained within two Pvu II cleavage sites thereon, which plasmid is capable of replication in an *E. coli* bacterial host species and contains expressible heterologous DNA coding for the crystal protein of *Bacillus thuringiensis* and includes an expression mechanism for said heterologous DNA which is recognized by the *E. coli* bacterial host species system but does not exhibit substantial growth phase limitations in the *E. coli* bacterial host species.

2. A plasmid as claimed in claim 1, wherein said plasmid fraction is derived from *B. thuringiensis* subspecies *tolworthi;* subspecies *darmstadiensis;* subspecies *sotto;* subspecies *thuringiensis,* F-10; subspecies *thuringiensis,* HD-290; subspecies *thuringiensis,* HD-120; subspecies *thuringiensis,* HD-2; subspecies *kurstaki,* HD-244; subspecies *kurstaki,* HD-73; subspecies *kurstaki,* HD-1; subspecies *alesti,* HD-4; subspecies *toumanoffi,* F-9; subspecies *galleriae,* HD-8; subspecies *wuhnanesis,* F-6; or subspecies *morrisoni*, F-5.

3. A plasmid as claimed in claim 1 or 2 including a DNA portion having substantial sequence homology to plasmid pBR322.

4. A plasmid chosen from plasmid pES1 (obtainable from ATCC Number 31995), pJWK20 (obtainable from ATCC Number 31997), or pJWK18 (obtainable from ATCC Number 31998), or a mutant plasmid having the *B. thuringiensis* crystal protein expressible coding characteristics of said plasmids pES1 or pJWK20 or pJWK18 in a bacterial host to which it is non-indigenous.

5. An *En coli* bacterial strain containing a plasmid as claimed in any one of claims 1 to 3 or a plasmid or mutant plasmid as claimed in claim 4.

6. A strain chosen from the bacterial strains *E. coli* ES12 (ATCC Number 31995), JWK1 (ATCC Number 31997) and JWK11 (ATCC Number 31998), in each case carrying a plasmid as claimed in claim 1 and *B. thuringiensis* crystal protein antigen-producing mutants thereof.

7. A genetically engineered *E. coli* bacterial strain which has been transformed by introduction of a plasmid according to claim 1.

8. A method of producing a plasmid as claimed in any one of claims 1 to 4 which comprises isolating from a plasmid fraction of a crystal protein-producing strain of *Bacillus thuringiensis,* which fraction is of molecular mass greater than $10 \times 10^6$ $M_r$, a fragment of DNA comprising an expressible DNA coding for the crystal protein, said fragment being identifiable with a DNA probe from plasmid pES1 (obtainable from ATCC Number 31995) which probe is contained within two Pvu II cleavage sites thereon, and ligating said DNA fragment with DNA of a plasmid capable of replication in an *E. coli* bacterial species whereby the correct arrangement for expression of the heterologous crystal protein-producing DNA coding in said *E. coli* bacterial host is achieved with the expression mechanism recognized by the host without exhibiting substantial growth phase limitations.

9. A method of creating a genetically engineered *E. coli* bacterial strain which is capable of producing the crystal protein of *Bacillus thuringiensis* comprising transforming said *E. coli* host bacterial strain by introducing thereinto a plasmid as claimed in any one of claims 1 to 3, or a plasmid or mutant plasmid as claimed in claim 4, whereby the expressible genetic material in the host includes heterologous DNA coding for the crystal protein of *B. thuringiensis* and identifiable with a DNA probe from plasmid pES1 (obtainable from ATCC Number 31995) and contained with two Pvu II cleavage sites thereon.

10. A method of preparing *Bacillus thuringiensis* crystal protein comprising growing an *E. coli* bacterial strain as claimed in any one of claims 5 to 7 or which has been created by a method as claimed in claim 9.

11. A method of producing an insecticidal effect in an environment which comprises either effecting a method as claimed in claim 11 and treating the environment with the resulting protein or exposing the environment to growth of a bacterial strain as claimed in claim 5 or claim 6 or to growth of a genetically engineered bacterial strain as claimed in claim 7.

**Claims for the Contracting State: AT**

1. A method of producing a plasmid capable of replication in an *E. coli* bacterial host species and containing expressible heterologous DNA coding for the crystal protein of *Bacillus thuringiensis,* which method comprises isolating from a plasmid fraction of a crystal protein-producing strain of *Bacillus thuringiensis,* which fraction is of molecular mass greater than $10 \times 10^6$ $M_r$, a fragment of DNA comprising as expressible DNA coding for the crystal protein and identifiable with a DNA fragment probe derived from plasmid pES1 (obtainable from ATCC Number 31995) which probe is contained within two Pvu II cleavage sites thereon, and ligating said DNA fragment with DNA of a plasmid capable of replication in an *E. coli* bacterial species whereby the correct arrangement for expression of the heterologous crystal protein-producing DNA coding in said *E. coli* bacterial host is achieved with the expression mechanism recognized by the host without exhibiting substantial growth phase limitations.

2. A method as claimed in claim 1, wherein said strain of *Bacillus thuringiensis* is *B. thuringiensis* subspecies *tolworthi;* subspecies *darmstadiensis;* subspecies *sotto;* subspecies thuringiensis, F-10; subspecies *thuringiensis,* HD-290; subspecies *thuringiensis,* HD-120; subspecies *thuringiensis,* HD-2; subspecies *kurstaki,* HD-244; subspecies *kurstaki,* HD-73; subspecies *Kurstaki,* HD-1; subspecies *alesti,* HD-4; subspecies *toumanoffi,* F-9; subspecies *galleriae,* HD-8; subspecies *wuhnanesis,* F-6; or subspecies *morrisoni,* F-5.

3. A method as claimed in claim 1 or claim 2, wherein the thus produced plasmid includes a DNA portion having substantial sequence homology to plasmid pBR322.

4. A method of creating a genetically engineered *E. coli* bacterial strain which is capable of producing the crystal protein of *Bacillus thuringiensis* comprising transforming a host *E. coli* bacterial strain by introducing thereinto a plasmid which has been produced by a method as claimed in any one of claims 1 to 3 or a descendent thereof retaining said expressible heterologous DNA coding for the crystal protein of *Bacillus thuringiensis* whereby the expressible genetic material in the host includes heterologous DNA coding for the crystal protein of *B thuringiensis.*

5. A method of preparing *Bacillus thuringiensis* crystal protein comprising growing a bacterial strain which has been created by a method as claimed in claim 4 or growing *E. coli* ES12 (ATCC Number 31995), JWK1 (ATCC Number 31997) or JWK11 (ATCC Number 31998).

6. A method of producing an insecticidal effect in an environment which comprises effecting a method as claimed in claim 5 and treating the environment with the resulting crystal protein, said crystal protein optionally being in the form of a formulation including a diluent, carrier or excipient, or exposing the environment to growth of a bacterial strain as defined in claim 4 or to growth of *E. coli* ES12 (ATCC Number 31995), JWK1 (ATCC Number 31997) or JWK11 (ATCC Number 31998).

**Patentansprüche für die Vertragsstaaten: BE CH DE FR GB IT LI LU NL SE**

1. Plasmid, enthaltend eine DNA, die von einer Plasmidfraktion von *B. thuringiensis* einer Molekularmasse größer als $10 \times 10^6$ $M_r$ abgeleitet ist und mit einer DNA-Fragmentsonde identifizierbar ist, die abgeleitet ist von Plasmid pES1 (erhältlich von ATCC Nr. 31995), wobei die Sonde innerhalb von zwei darauf befindlichen *Pvu* II-Schnittstellen enthalten ist und das Plasmid zur Replikation in einer *E. coli*-Wirtsbakterienspezies fähig ist und expressible, heterologe DNA enthält, die für das Kristallprotein von *Bacillus thuringiensis* kodiert und einen Expressionsmechanismus für diese heterologe DNA enthält, der durch das Wirtsbakterienspezies-System erkannt wird, aber keine wesentlichen Wachstumsphasenbegrenzungen in der *E. coli*-Wirtsbakterienspezies zeigt.

2. Plasmid nach Anspruch 1, dadurch gekennzeichnet, daß die Plasmidfraktion abgeleitet ist von *B. thuringiensis* Subspezies *tolworthi,* Subspezies *darmstadiensis;* Subspezies *sotto;* Subspezies *thuringiensis,* F-10; Subspezies *thuringiensis,* HD-290; Subspezies *thuringiensis,* DH-120; Subspezies *thuringiensis,* HD-2; Subspezies *kurstaki,* HD-244; Subspezies *kurstaki,* HD-73; Subspezies *kurstaki,* HD-1; Subspezies *alesti,* HD-4; Subspezies *toumanoffi,* F-9; Subspezies *galleriae,* HD-8; Subspezies *wuhnanesis,* F-6 oder Subspezies *morrisoni,* F-5.

3. Plasmid nach Anspruch 1 oder 2, dadurch gekennzeichnet, daß es einen DNA-Teil enthält, der im wesentlichen eine Sequenzhomologie zum Plasmid pBR322 besitzt.

4. Plasmid, ausgewählt aus Plasmid pES1 (erhätlich von ATCC Nr. 31995), pJWK20 (erhältlich von ATCC Nr. 31997) oder pJWK18 (erhältlich von ATCC Nr. 31998) oder einem Mutanten-Plasmid, das die Kodierungscharakteristika der Plasmide pES1 oder pJWK20 oder pJWK18 zur Expression des *B. thuringiensis*-Kristallproteins besitzt, in einem Wirksbakterium, in dem es nicht entstanden ist.

5. *E. coli*-Bakterienstamm, enthaltend ein Plasmid nach einem der Ansprüche 1 bis 3 oder ein Plasmid oder Mutanten-Plasmid nach Anspruch 4.

6. Stamm, ausgewählt aus den Bakterienstämmen *E. coli* ES12 (ATCC Nr. 31995), JWK1 (ATCC Nr. 31997) und JWK11 (ATCC Nr. 31998), der jeweils ein Plasmid nach Anspruch 1 enthält und *B. thuringiensis*-Krisallprotein-Antigen produzierende Mutanten davon.

7. Gentechnologisch gebildeter *E. coli*-Bakterieinstamm, der durch Einführung eines Plasmids gemäß Anspruch 1 transformiert worden ist.

8. Verfahren zur Herstellung eines Plasmids nach einem der Ansprüche 1 bis 4, dadurch gekennzeichnet, daß man aus einer Plasmidfraktion eines ein Kristallprotein produzierenden Stammes von *Bacillus thuringiensis,* wobei die Fraktion eine Molekularmasse größer als $10 \times 10^6$ $M_r$ besitzt, ein DNA-Fragment isoliert, das eine expressible DNA enthält, die für das Kristallprotein kodiert und mit einer DNA-Sonde von Plasmid pES1 (erhältlich von ATCC Nr. 31995) identifizierbar ist, wobei die Sonde innerhalb von zwei darauf befindlichen *Pvu* II-Schnittstellen enthalten ist und Ligierung des DNA-Fragments mit DNA eines zur Replikation in einer *E. coli*-Bakterienspezies fähigen Plasmids, wobei die korrekte Anordnung zur Expression der heterologen, das Kristallprotein produzierenden DNA, die in diesem *E. coli*-Bakterienwirt kodiert, mit dem Expressionsmechanismus erreicht wird, der durch den Wirt erkannt wird, ohne wesentliche Wachstumsphasen-Begrenzungen zu zeigen.

9. Verfahren zur Erzeugung eines gentechnologisch gebildeten *E. coli*-Bakterienstammes, der zur Produktion des Kristallproteins von *Bacillus thuringiensis* fähig ist, dadurch gekennzeichnet, daß man einen *E. coli*-Wirtsbakterienstamm durch Einbringen eines Plasmids nach einem der Ansprüche 1 bis 3 oder einem Plasmid oder Mutanten-Plasmid nach Anspruch 4 in diesen Stamm transformiert, wobei das expressible genetische Material in dem Wirt heterologe DNA enthält, die für das Kristallprotein von *B. thuringiensis* kodiert und identifizierbar ist mit einer DNA-Sonde von Plasmid pES1 (erhältlich von ATCC Nr. 31995), die innerhalb von zwei darauf befindlichen *Pvu* II-Schnittstellen enthalten ist.

10. Verfahren zur Herstellung von *Bacillus thuringiensis*-Kristallprotein, dadurch gekennzeichnet, daß man einen in einem der Ansprüche 5 bis 7 beanspruchten oder nach einem in Anspruch 9 beanspruchten Verfahren gebildeten *E. coli*-Bakterienstamm wachsen läßt.

11. Verfahren zur Erzeugung einer insektiziden Wirkung in einer Umgebung, dadurch gekennzeichnet, daß man ein in Anspruch 10 beanspruchtes Verfahren durch führt und die Umgebung mit dem gebildeten Kristallprotein behandelt, oder die Umgebung dem Wachstum eines in Anspruch 5 oder Anspruch 6 beanspruchten Bakterienstammes aussetzt oder dem Wachstum eines in Anspruch 7 beanspruchten gentechnologisch gebildeten Bakterienstammes.

**Patentansprüche für den Vertragsstaat: AT**

1. Verfahren zur Herstellung eines zur Replikation in einer *E. coli*-Wirtsbakterienspezies fähigen Plasmids, das eine expressible heterologe DNA enthält, die für das Kristallprotein von *Bacillus thuringiensis* kodiert, dadurch gekennzeichnet, daß man aus einer Plasmidfraktion eines ein Kristallprotein produzierenden Stammes von *Bacillus thuringiensis,* wobei die Fraktion eine Molekularmasse größer als $10 \times 10^6$ $M_r$ besitzt, ein DNA-Fragment isoliert, das eine expressible DNA enthält, die für das Kristallprotein kodiert und mit einer DNA-Fragmentsonde identifizierbar ist, die von einem pES1-Plasmid (erhältlich von ATCC Nr. 31995) abgeleitet ist, wobei die Sonde innerhalb von zwei darauf befindlichen *Pvu* II-Schnittstellen enthalten ist, und Ligierung des DNA-Fragments mit DNA eines zur Replikation in einer *E. coli*-Bakterienspezies fähigen Plasmids, wobei die korrekte Anordnung zur Expression der heterologen Kristallprotein produzierenden DNA, die in diesem *E. coli*-Wirtsbakterium kodiert, mit dem Expressionsmechanismus erreicht wird, der durch den Wirt erkannt wird, ohne wesentliche Wachstumsphasenbegrenzungen zu zeigen.

2. Verfahren nach Anspruch 1, dadurch gekennzeichnet, daß der *Bacillus thuringiensis*-Stamm die *B. thuringiensis* Subspezies *tolworthi*; die Subspezies *darmstadiensis;* Subspezies *sotto;* Subspezies *thuringiensis* F-10; Subspezies *thuringiensis,* HD-290; Subspezies *thuringiensis,* HD-120; Subspezies *thuringiensis,* HD-2; Subspezies *kurstaki,* HD-244; Subspezies *kurstaki,* HD-73; Subspezies *kurstaki,* HD-1; Subspezies *alesti,* HD-4; Subspezies *toumanoffi,* F-9; Subspezies *galleriae,* HD-8; Subspezies *wuhnanesis,* F-6 oder Subspezies *morrisoni,* F-5 ist.

3. Verfahren nach Anspruch 1 oder 2, dadurch gekennzeichnet, daß das so hergestellte Plasmid einen DNA-Teil enthält, der eine wesentliche Sequenzhomologie zum Plasmid pBR322 besitzt.

4. Verfahren zur Erzeugung eines gentechnologisch gebildeten *E. coli*-Bakterienstammes, der zur Produktion des Kristallproteins von *Bacillus thuringiensis* fähig ist, dadurch gekennzeichnet, daß man einen *E. coli*-Wirtsbakterienstamm transformiert durch Einbringen eines Plasmids in diesen Stamm, das nach in einem der Ansprüche 1 bis 3 beanspruchten Verfahren hergestellt worden ist, oder eines Abkömmlings davon, der die expressible heterologe DNA, die für das Kristallprotein von *Bacillus thuringiensis* kodiert, beibehält, wobei das expressible genetische Material in dem Wirt heterologe DNA enthält, die für das Kristallprotein von *B. thuringiensis* kodiert.

5. Verfahren zur Herstellung von *Bacillus thuringiensis*-Kristallprotein, dadurch gekennzeichnet, daß man einen Bakterienstamm, der nach einem in Anspruch 4 beanspruchten Verfahren erzeugt wurde, wachsen läßt oder *E. coli* ES12 (ATCC Nr. 31995), JWK1 (ATCC Nr. 31997) oder JWK11 (ATCC Nr. 31998) wachsen läßt.

6. Verfahren zur Erzeugung einer insektiziden Wirkung in einer Umgebung, dadurch gekennzeichnet, daß man ein in Anspruch 5 beanspruchtes Verfahren durchführt und die Umgebung mit dem gebildeten Kristallprotein behandelt, wobei das Kristallprotein gegebenenfalls als Formulierung vorliegt, die ein Verdünnungsmittel, einen Träger oder ein Vehikel enthält oder die Umgebung dem Wachstum eines wie in Anspruch 4 definierten Bakterienstamm aussetzt, oder dam Wachstum von *E. coli* ES12 (ATCC Nr. 31995), JWK1 (ATCC Nr. 31997) oder JWK11 (ATCC Nr 31998).

**Revendications pour les Etats Contractants: BE CH DE FR GB IT LI LU NL SE**

1. Un plasmide contenant de l'ADN dérivé d'une fraction plasmidique de *B. thuringiensis* de masse moléculaire supérieure à $10 \times 10^6$ $M_r$ et identifiable avec une sonde de fragment d'ADN dérivée du plasmide pES1 (que l'on peut se procurer sous le numéro ATCC 31995), laquelle sonde est contenue à l'intérieur de deux sites de clivage *Pvu* II de celui-ci, lequel plasmide est capable de réplication dans une espèce bactérienne hôte *E. coli* et contient de l'ADN hétérologue exprimable codant pour la protéine cristalline de *Bacillus thuringiensis* et comprend un mécanisme d'expression pour ledit ADN hétérologue qui est reconnu par le système de l'espèce bactérienne hôte *E. coli* mais ne présente pas de limitations notables de la phase de croissance dans l'espèce bactérienne hôte *E. coli.*

2. Un plasmide comme revendiqué dans la revendication 1 dans lequel ladite fraction plasmidique dérive de *B. thuringiensis* sous-espèce *tolworthii;* sous-espèce *darmstadiensis;* sous-espèce *sotto;* sous-expèce *thuringiensis* F-10; sous-espèce *thuringiensis* HD-290; sous-espèce *thuringiensis* HD-120; sous-espèce *thuringiensis* HD-2; sous-espèce *kurstaki* HD-244; sous-espèce *kurstaki* HD-73; sous-espèce *kurstaki* HD-1; sous-espèce *alesti* HD-4; sous-espèce *toumanoffi* F-9; sous-espèce *galleriae* HD-8; sous-espèce *wuhnanesis* F-6 ou sous-espèce *morrisoni* F-5.

3. Un plasmide comme revendiqué dans la revendication 1 ou 2 comprenant une portion d'ADN ayant une homologie notable de séquence avec le plasmide pBR322.

4. Un plasmide choisi parmi le plasmide pES1 (que l'on peut se procurer sous le numéro ATCC 31995), pJWK20 (que l'on peut se procurer sous le numéro ATCC 31997) ou pJWK18 (que l'on peut so procurer sous le numéro ATCC 31998) ou un plasmide mutant ayant les caractéristiques de codage exprimables de la protéine cristalline de *B. thuringiensis* desdits plasmides pES1 ou pJWK20 ou pJWK18 dans un hôte bactérien dont il n'est pas originaire.

5. Une souche bactérienne d'*E. coli* contenant un plasmide comme revendiqué dans l'une quelconque des revendications 1 à 3 ou un plasmide ou plasmide mutant comme revendiqué dans la revendication 4.

6. Une souche choisie parmi les souches bactériennes *E. coli* ES12 (numéro ATCC 31995), JWK1 (numéro ATCC 31997) et JWK11 (numéro ATCC 31998) portant dans chaque cas un plasmide comme revendiqué dans la revendication 1 et les mutants producteurs de l'antigène protéine cristalline de *B. thuringiensis* de celle-ci.

7. Une souche bactérienne d'*E. coli* de génie génétique qui a été transformée par introduction d'un plasmide selon la revendication 1.

8. Un procédé pour produire un plasmide comme revendiqué dans l'une quelconque des revendications 1 à 4 qui comprend l'isolement d'une fraction plasmidique d'une souche de *Bacillus thuringiensis* productrice de protéine cristalline, laquelle fraction a une masse moléculaire supérieure à $10 \times 10^6$ $M_r$, un fragment d'ADN comprenant un ADN exprimable codant pour la protéine cristalline, ledit fragment étant identifiable avec une sonde d'ADN du plasmide pES1 (que l'on peut se procurer soud le numéro ATCC 31995), laquelle sonde est contenue à l'intérieur de deux sites de clivage *Pvu* II de celui-ci, et ligation dudit fragment d'ADN avec l'ADN d'un plasmide capable de réplication dans une espèce bactérienne d'*E. coli,* si bien que l'arrangement convenable pour l'expression de l'ADN hétérologue produisant la protéine cristalline codant dans ledit hôte bactérien d'*E. coli* est obtenu avec le mécanisme d'expression reconnu par l'hôte sans qu'il y ait de limitations notables de la phase de croissance.

9. Un procédé pour créer une couche bactérienne d'*E. coli* de génie génétique capable de produire la protéine cristalline de *Bacillus thuringiensis* comprenant la transformation de ladite souche bactérienne hôte *E. coli* par introduction dans celle-ci d'un plasmide comme revendiqué dans l'une quelconque des revendications 1 à 3 ou d'un plasmide ou plasmide mutant comme revendiqué dans la revendication 4 pour que le matériel génétique exprimable dans l'hôte comprenne l'ADN hétérologue codant pour la protéine cristalline de *B. thuringiensis* et identifiable avec une sonde d'ADN du plasmide pES1 (que l'on peut se procurer sous le numéro ATCC 31995) et contenu à l'intérieur de deux sites de clivage *Pvu* II de celui ci.

10. Un procédé de préparation de la protéine cristalline de *Bacillus thuringiensis* comprenant la culture d'une souche bactérienne d'*E. coli* comme revendiqué dans l'une quelconque des revendication 5 à 7 ou qui a été créée selon un procédé comme revendiqué dans la revendication 9.

11. Un procédé de production d'un effet insecticide dans un environnement qui comporte soit la mise en pratique d'un procédé comme revendiqué dans la revendication 10 et le traitement de l'environnement avec la protéine obtenus, soit l'exposition de l'environnement à la croissance d'une souche bactérienne comme revendiquée dans la revendication 5 ou la revendication 6, soit la culture d'une souche bactérienne de génie génétique comme revendiquée dans la revendication 7.

**Revendications pour l'Etat Contractant: AT**

1. Un procédé de production d'un plasmide capable de réplication dans une espèce bactérienne hôte *E. coli* et contenant de l'ADN hétérologue exprimable codant pour la protéine cristalline de *Bacillus thuringiensis,* lequel procédé comprend l'isolement à partir d'une fraction plasmidique d'une souche de *Bacillus thuringiensis* productrice de protéine cristalline, ladite fraction ayant une masse moléculaire supérieure à $10 \times 10^{-6}$ $M_r$, d'un fragment d'ADN comprenant un ADN exprimable codant pour la protéine cristalline et identifable avec une sonde constituée d'un fragment d'ADN dérivant du plasmide pES1 (que l'on peut se procurer sous le numéro ATCC 31995), laquelle sonde est contenue à l'intérieur de deux sites de clivage *Pvu* II de celui-ci, et la ligation dudit fragment d'ADN avec l'ADN d'un plasmide capable de réplication dans une espèce bactérienne d'*E. coli* pour obtenir l'arrangement convenant à l'expression de l'ADN hétérologue produisant la protéine cristalline codant dans ledit hôte bactérien d'*E. coli* avec le mécanisme d'expression reconnu par l'hôte sans qu'il y ait de limitations notables de la phase de croissance.

2. Un procédé comme revendiqué dans la revendication 1 dans lequel ladite souche de *Bacillus thuringiensis* est *B. thuringiensis* sous-espèce *tolworthii;* sous-espèce *darmstadiensis;* sous-espèce *sotto;* sous-espèce *thuringiensis* F-10; sous-espèce *thuringiensis* HD-290; sous-espèce *thuringiensis* HD-120; sous-espèce *thuringiensis* HD-2; sous-espèce *kurstaki* HD-244; sous-espèce *kurstaki* HD-73; sous-espèce *kurstaki* HD-1; sous-espèce *alesti* HD-4; sous-expèce *toumanoffi* F-9; sous-expèce *galleriae* HD-8; sous-espèce *wuhnanesis* F-6 ou sous-espèce *morrisoni* F-5.

3. Un procédé comme revendiqué dans la revendication 1 ou la revendication 2 dans lequel le plasmide ainsi produit comprend une portion d'ADN ayant une homologie notable de séquence avec le plasmide pBR322.

4. Un procédé pour créer une souche bactérienne d'*E. coli* de génie génétique capable de produite la protéine cristalline de *Bacillus thuringiensis* comprenant la transformation d'une souche batérienne hôte *E. coli* par introduction dans celle-ci d'un plasmide qui a été produit selon un procédé comme revendiqué dans l'une quelconque des revendications 1 à 3 ou un descendant de celui-ci conservant ledit ADN hétérologue exprimable codant pour la protéine cristalline de *Bacillus thuringiensis* pour que le matériel génétique exprimable dans l'hôte comprenne l'ADN hétérologue codant pour la protéine cristalline de *B. thuringiensis.*

5. Un procédé de préparation de la protéine cristalline de *Bacillus thuringiensis* comprenant la culture d'une souche bactérienne qui a été créée seoon un procédé comme revendiqué dans la revendication 4 ou la culture d'*E. coli* ES12 (numéro ATCC 31995), JWK1 (numéro ATCC 31997) ou JWK11 (numéro ATCC 31998).

6. Un procédé de production d'un effet insecticide dans un environnement qui comporte la mise en pratique d'un procédé comme revendiqué dans la revendication 5 et le traitement de l'environnement avec la protéine cristalline obtenue, ladite protéine cristalline étant éventuellement sous forme d'une composition comprenant un diluant, un support ou un excipient ou l'exposition de l'environnement à la culture d'une souche bactérienne comme définie dans la revendication 4 ou à la culture d'*E. coli* ES12 (numéro ATCC 31995), JWK1 (numéro ATCC 31997) ou JWK11 (numéro ATCC 31998).

FIG. 1

FIG. 2

0 063 949

FIG. 3

FIG. 4

2